(19) Europäisches Patentamt
European Patent Office
Office européen des brevets

(11) EP 3 485 726 B1

(12) EUROPEAN PATENT SPECIFICATION

(45) Date of publication and mention of the grant of the patent:
**25.03.2026 Bulletin 2026/13**

(21) Application number: **17827898.2**

(22) Date of filing: **10.07.2017**

(51) International Patent Classification (IPC):
***A01H 4/00*** *(2006.01)* ***A01H 5/10*** *(2018.01)*
***C12N 5/00*** *(2006.01)* ***C12N 5/04*** *(2006.01)*
***A01H 6/54*** *(2018.01)*

(52) Cooperative Patent Classification (CPC):
**A01H 4/005; A01H 4/002; A01H 6/54; A01H 6/542**

(86) International application number:
**PCT/KR2017/007356**

(87) International publication number:
**WO 2018/012827 (18.01.2018 Gazette 2018/03)**

(54) **CULTURING METHOD FOR CULTURED LEGUMINOUS ROOTS HAVING INCREASED COUMESTROL CONTENT**

ANBAUVERFAHREN FÜR ANGEBAUTE LEGUMINOSENWURZELN MIT ERHÖHTEM COUMESTROLGEHALT

PROCÉDÉ DE CULTURE POUR RACINES DE LÉGUMINEUSES CULTIVÉES À TENEUR ACCRUE EN COUMESTROL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.07.2016 KR 20160089464**
**29.06.2017 KR 20170082586**

(43) Date of publication of application:
**22.05.2019 Bulletin 2019/21**

(73) Proprietors:
- **Amorepacific Corporation**
  **Seoul 04386 (KR)**
- **Chungbuk National University Industry-Academic Cooperation Foundation**
  **Cheongju-si, Chungcheongbuk-do 28644 (KR)**

(72) Inventors:
- **LEE, Eun Jung**
  **Yongin-si**
  **Gyeonggi-do 17074 (KR)**
- **KANG, Young-Gyu**
  **Yongin-si**
  **Gyeonggi-do 17074 (KR)**
- **PARK, Jun Seong**
  **Yongin-si**
  **Gyeonggi-do 17074 (KR)**
- **PARK, So Young**
  **Cheongju-si**
  **Chungcheongbuk-do 28385 (KR)**
- **KIM, Young Eun**
  **Sejong 30101 (KR)**

(74) Representative: **Novagraaf Technologies**
**2 rue Sarah Bernhardt**
**CS90017**
**92665 Asnières-sur-Seine Cedex (FR)**

(56) References cited:
**KR-A- 20010 070 932** **KR-A- 20130 079 220**
**KR-A- 20140 060 201** **KR-A- 20140 089 691**

- **BASKARAN P ET AL: "Psoralen production in hairy roots and adventitious roots cultures of Psoralea coryfolia", BIOTECHNOLOGY LETTERS, SPRINGER NETHERLANDS, DORDRECHT, vol. 31, no. 7, 7 March 2009 (2009-03-07), pages 1073 - 1077, XP019670338, ISSN: 1573-6776**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- KEON SOO HA ET AL: "Adventitious root formation from cotyledon in soybean (Glycine max L.) cultivars", KOREAN J. PLANT BIOTECHNOLOGY, vol. 29, no. 1, 1 January 2002 (2002-01-01), pages 31 - 36, XP055649080, Retrieved from the Internet <URL:http://koreascience.or.kr/article/JAKO200211921614692.page> [retrieved on 20191204]
- AHMAD NASEEM ET AL: "Effect of PGRs in adventitious root culture in vitro: present scenario and future prospects", RENDICONTI LINCEI. SCIENZE FISICHE E NATURALI, SPRINGER ITALIA SRL, MILAN, vol. 26, no. 3, 23 June 2015 (2015-06-23), pages 307 - 321, XP035533613, ISSN: 2037-4631, [retrieved on 20150623], DOI: 10.1007/S12210-015-0445-Y
- YUN SUN LEE ET AL: "Induction and Proliferation of Adventitious Roots from 'Aloe vera' Leaf Tissues for 'in vitro' Production of Aloe-emodin", PLANT OMICS, 1 July 2011 (2011-07-01), pages 190 - 194, XP055649381, Retrieved from the Internet <URL:https://www.pomics.com/yang_4_4_2011_190_194.pdf> [retrieved on 20191204]
- MAGDALENA W�JCIAK-KOSIOR ET AL: "The Stimulatory Effect of Strontium Ions on Phytoestrogens Content in Glycine max (L.) Merr", MOLECULES, vol. 21, no. 1, 14 January 2016 (2016-01-14), pages 90, XP055649397, DOI: 10.3390/molecules21010090
- MAZUR, W. M. ET AL.: "Isoflavonoids and lignans in legumes: Nutritional and health aspects in humans", NUTRITIONAL BIOCHEMISTRY, vol. 9, 1 January 1998 (1998-01-01), pages 193 - 200
- BOURGAUD, F. ET AL.: "Production of Flavonoids by Psoralea Hairy Root Cultures", PLANT CELL , TISSUE AND ORGAN CULTURE, vol. 56, 1999, pages 97 - 104, XP055273987
- HABIBI, PEYMAN ET AL.: "Efficient Genetic Trans Formation and Regeneration System from Hairy Root of Origanum Vulgate", PHYSIOLOGY AND MOLECULAR BIOLOGY OF PLANTS, (ELECTRONIC PUBLISHING, vol. 22, no. 2, 30 April 2016 (2016-04-30), pages 271 - 277, XP035996919

## Description

[Technical Field]

The present disclosure relates to a culturing method for cultured leguminous roots having increased coumestrol content.

[Background Art]

To date, coumestrol has been known as the strongest substance among other vegetable estrogens. It is mainly found in seeds, roots, and leaves of leguminosae and compositae plants, and is generally classified as coumestan series compound and is a type of isoflavonoid. The coumestrol has come to attention due to the fact that it is secreted at a high concentration in an injured site when a plant is damaged, and has anti-fungal and anti-viral activities through anti-oxidative, anti-inflammatory, and anti-toxic activities to prevent infection. The reason for this is that infection due to various bacteria, fungi and viruses induces the synthesis of various aromatic compounds including coumestrol. Such antibiotic activity of coumestrol is based on its phenolic structure, which is a chemical framework for an antioxidant, and thus the introduction of free radical oxidants is inhibited and the production of peroxide in body is blocked. Furthermore, only coumestrol is known to have an estrogen effect among many other natural coumestan derivatives. An estrogen effect was evaluated based on a change in weight of uterus after the oral administration of coumestrol to an immature mouse. From the test results, it was observed that coumestrol exhibits an effective estrogen effect in young female mice, but has no estrogen effect in mature male mice. Also, it was demonstrated that coumestrol has no toxicity.

Currently, commercial natural coumestrol is highly expensive since coumestrol is present in a very small amount in leguminosae plants. For this reason, there were attempts to obtain coumestrol through synthesis. However, an approach to develop a simple synthesis method for coumestrol is yet to be found due to a complicated chemical structure wherein a number of aromatic rings are fused. Although several synthesis methods have been reported, each method has a variety of problems and thus makes commercialization challenging. Moreover, natural components are considered to be better in safety than chemically synthesized components, so a method for mass-production from natural components is being developed.

Document BASKARAN P ET AL: "Psoralen production in hairy roots and adventitious roots cultures of Psoralea coryfolia", BIOTECHNOLOGY LETTERS, SPRINGER NETHERLANDS, DORDRECHT, vol.531, no. 7, 7 March 2009 (2009-03-07), pages 1073-1077 disloses production of adventitious and hary root cultures of *Psoralea coryfolia.* The plant belongs to the family *Fabaceae* and is disclosed to produce coumesterol in it roots, notably from document BOURGAUD, F. ET AL: "Production of Flavonoids by Psoralea Hairy Root Cultures", PLANT CELL, TISSUE AND ORGAN CULTURE, vol. 56, 1999, pages 97-104.

Document Keon Soo Ha ET AL: "Adventitious root formation from cotyledon in soybean (Glycine max L.) cultivars", Korean J. Plant Biotechnology, vol. 29, no. 1 1 January 2002, pages 31-36 and document KR 2014 0060201 A (BIO-FD&C) 19 mai 2014 disclose production of adventitious roots from soybean.

Document AHMAD NASEEM ET AL: "Effect of PGRs in adventitious root culture in vitro: present scenario and future prospects", RENDICONTI LINCEI. SCIENZE FISICHE E NATURALI, SPRINGER ITALIA SRL, MILAN, vol. 26, no. 3, 23 juin 2015 (2015-06-23), pages 307-321 discloses the effect of different plant growth regulators (PGRs) in adventitious root culture in vitro.

Document Yun Sun Lee ET AL: "Induction and Proliferation of Adventitious Roots from ’Aloe vera’ Leaf Tissues for ’in vitro’ Production of Aloe-emodin", Plant Omics, 1 July 2011 (2011-07-01), pages 190-194 discloses induction and proliferation of adventitious roots from *Aloe vera* leaf tissues for *in vitro* production of aloe-emodin.

Document MAGDALENA WÓJCIAK-KOSIOR ET AL: "The Stimulatory Effect of Strontium Ions on Phytoestrogens Content in Glycine max (L.) Merr", MOLECULES, vol. 21, no. 1, January 14, 2016, page 90, discloses the stimulatory effect of Strontium Ions on phytoestrogens content in *Glycine max* (L.)

Therefore, the inventors have studied a method for naturally mass-producing coumestrol and have completed the present invention.

[Summary of Invention]

[Technical Problem]

To solve the aforementioned issues, the present inventors have completed the present invention relating to a culturing method for cultured leguminous roots having increased coumestrol content. The method is as defined in annexed claims 1-5.

According to an aspect of the present invention, there is provided a culturing method for cultured leguminous roots having increased coumestrol content.

According to an aspect of the present invention, there is provided a culturing method for cultured leguminous roots having increased coumestrol content which is capable of producing a constant amount of coumestrol throughout the year.

According to an aspect of the present invention, there is provided a culturing method for cultured leguminous roots having increased coumestrol content which is capable of mass-producing coumestrol.

[Solution to Problem]

According to an aspect, the present invention provides a culturing method for cultured leguminous roots having increased coumestrol content comprising, among the other features defined in annexed claim 1, the steps of:

(a) germinating leguminous seeds in a culture medium to induce *in vitro* plants having cotyledons, hypocotyls, and radicles;

(b) culturing, in a culture medium, at least one site of cotyledons, hypocotyls, and radicles of the induced *in vitro* plant to induce site-specific cultured roots; and

(c) multiplying the induced site-specific cultured roots in a culture medium,

wherein the culture medium contains nutrient components of $NH_4NO_3$, $CaCl_2 \cdot 2H_2O$, $MgSO_4 \cdot 7H_2O$, $KH_2PO_4$, and $KNO_3$.

According to an aspect, the concentration of $NH_4NO_3$ in the culture medium may be between 1,500 and 2,000 mg/L, the concentration of $CaCl_2 \cdot 2H_2O$ in the culture medium may be between 300 and 500 mg/L, the concentration of $MgSO_4 \cdot 7H_2O$ in the culture medium may be between 300 and 500 mg/L, the concentration of $KH_2PO_4$ in the culture medium may be between 100 and 200 mg/L, and the concentration of $KNO_3$ in the culture medium may be between 1,700 and 2,100 mg/L.

According to the method disclosed therein, the culture medium may be a MS medium (Murashige and Skoog medium).

According to the method disclosed therein, in the step (a), the leguminous seeds is from Glycine Max Merr.

According to an aspect, in the step (a), the culture medium contains 10-100 g/L of sucrose based on a total volume of the medium.

According to the method disclosed therein, in the step (b), the culture medium contains at least one of IBA (indole butyric acid) and NAA (naphthalene acetic acid) at an amount between 0.1 to 10 mg/L based on the total volume of the medium, and the culture medium contains 10-100 g/L of sucrose based on the total volume of the medium.

According to an aspect, in the step (b), the culture medium contains 2 to 8 mg/L of IBA (indole butyric acid) based on the total volume of the medium.

According to an aspect, the leguminous seeds in the step (a) are from Glycine Max Merr; the induced site in the step (b) is at least one of hypocotyls and radicles; and the culture medium in the step (b) contains 3 to 5 mg/L of IBA (indole butyric acid) based on the total volume of the medium.

According to the method as defined in annexed claim 1, in the step (c), the culture medium contains 3 to 5 mg/L of IBA (indole butyric acid) and 10-100 g/L of sucrose based on the total volume of the medium.

According to an aspect, in the step (c), the culture medium is 0.5-1.5 MS medium and contains 30-60 g/L of sucrose.

[Advantageous Effects of Invention]

In an aspect, the culturing method according to the present invention is capable of mass-producing coumestrol.

In an aspect, the culturing method according to the present invention is capable of producing a constant amount of coumestrol throughout the year.

In an aspect, the culturing method according to the present invention is capable of producing a large amount of coumestrol in a short time.

In another aspect, the cultured leguminous roots have increased coumestrol content.

In another aspect, the cultured leguminous roots have uniform coumestrol content.

[Brief Description of Drawings]

Fig. 1 is a schematic view illustrating the germination of leguminous seeds and the induction of *in vitro* plant in accordance with Preparation Example 1-1.

Figs. 2 and 3 show the induction status after 2 weeks of culturing depending on the type of bean, the induced site of *in vitro* plant, and the type and amount of auxin, in accordance with Preparation Example 1-2.

Figs. 4 and 5 show the growth status of the cultured leguminous roots depending on the type of bean and the induced site, and the result of measuring the content of coumestrol in cultured leguminous roots, in accordance with Experimental Example 1.

Fig. 6 shows the result of an optimum medium composition for coumestrol multiplication in the step of multiplying cultured leguminous roots in the Preparation Example 1-3, as measured by a DOE method.

[Description of Embodiments]

Hereinafter, the present invention will be described in detail.

The coumestrol is a primary bioactive substance of soybean. It is present in various amounts in leguminous plant depending on its external environment as well as in largely differential amount depending on genus/species/variety thereof. Especially, it is present in a very small amount in a plant. When coumestrol is produced using cultured leguminous roots induced by the culturing method according to the present invention defined in claims 1-5, coumestrol can be produced at a constant amount throughout the year. Also, the mass-production of *in vitro* coumestrol can be achieved by a control of culture process.

According to an aspect, the present invention provides a culturing method for cultured leguminous roots having increased coumestrol content comprising, among the features of the method as defined in annexed claim 1, the steps of:

(a) germinating leguminous seeds in a culture medium to induce *in vitro* plants having cotyledons, hypocotyls, and radicles;

(b) culturing, in a culture medium, at least one site of cotyledons, hypocotyls, and radicles of the induced *in vitro* plant to induce site-specific cultured roots; and

(c) multiplying the induced site-specific cultured roots in a culture medium,

wherein the culture medium contains nutrient components of $NH_4NO_3$, $CaCl_2•2H_2O$, $MgSO_4•7H_2O$, $KH_2PO_4$, and $KNO_3$.

As used herein, "a leguminous plant" may be any leguminous plant which is capable of producing coumestrol.

As used herein, seeds of leguminous plant are seeds from Glycine max Merr.

In this specification, "a culturing method for cultured leguminous roots" refers to a method of extracting at least one of a specific cell, tissue and organ from leguminous plant Glycine max Merr. and culturing *in vitro* in a culture medium containing nutrients under an aseptic condition to regenerate callus or single cells as an organic or complete plant.

Such culturing method for cultured plant or leguminous roots may be called plant or leguminous plant explantation, tissue culture, *in vitro* culture, aseptic culture, or plant stem-cell culture.

According to an aspect, the concentration of $NH_4NO_3$ in the culture medium may be between 1,500 and 2,000 mg/L, the concentration of $CaCl_2•2H_2O$ in the culture medium may be between 300 and 500 mg/L, the concentration of $MgSO_4•7H_2O$ in the culture medium may be between 300 and 500 mg/L, the concentration of $KH_2PO_4$ in the culture medium may be between 100 and 200 mg/L, and the concentration of $KNO_3$ in the culture medium may be between 1,700 and 2,100 mg/L.

According to the method as defined in claim 1, the culture medium may be a MS medium (Murashige and Skoog medium).

Specifically, according to a concentration of inorganic substance in the medium, 1/4 MS, 1/2 MS, 3/4 MS, 1 MS, 3/2 MS or 2 MS medium may be used.

According to the method as defined in annexed claim 1, in the step (a), the culture medium contains 10-100 g/L of sucrose based on the total volume of the medium.

Specifically, the concentration of sucrose may be at least 10 g/L, at least 20 g/L, at least 21 g/L, at least 22 g/L, at least 23 g/L, at least 24 g/L, at least 25 g/L, at least 26 g/L, at least 27 g/L, at least 28 g/L, at least 29 g/L, or at least 30 g/L based on the total volume of the culture medium. Further, the concentration of sucrose may be 100 g/L or less, 80 g/L or less, 60 g/L or less, 50 g/L or less, 40 g/L or less, 39 g/L or less, 38 g/L or less, 37 g/L or less, 36 g/L or less, 35 g/L or less, 34 g/L or less, 33 g/L or less, 32 g/L or less, 31 g/L or less, or 30 g/L or less based on the total volume of the culture medium. When the concentration of sucrose in the step (a) is within the range indicated above, a high induction of *in vitro* plant can be achieved.

According to an aspect, the induction of *in vitro* plant in the step (a) may be carried out until cotyledons, hypocotyls, and radicles are generated from seeds. Such induction of *in vitro* plant in the step (a) may be carried out for 1 to 25 days. Specifically, the induction of *in vitro* plant in the step (a) may be carried out for at least 1 day, at least 1.5 days, at least 2 days, at least 2.5 days, at least 7 days, at least 8 days, at least 9 days, at least 10 days, at least 11 days, at least 12 days, at least 13 days, at least 14 days, or at least 15 days. Further, the induction may be carried out for 25 days or less, 24 days or less, 23 days or less, 22 days or less, 21 days or less, 20 days or less, 19 days or less, 18 days or less, 17 days or less or 16 days or less.

According to an aspect, the induction of *in vitro* plant in the step (a) may be carried out until the length of *in vitro* plant becomes 8 to 15cm. As used herein, "length of *in vitro* plant" refers to a straight length from an end of cotyledon to an end of radicle. Specifically, the length may be at least 8cm, at least 9cm, at least 10cm, or at least 11cm, and also may be 15cm or less, 14cm or less or 13cm or less.

According to an aspect, the induction of *in vitro* plant in the step (a) may be carried out under light condition.

According to the method as defined in annexed claim 1, in the step (b), the culture medium contains at least one of IBA (indole butyric acid) and NAA (naphthalene acetic acid) at an amount between 0.1 to 10 mg/L based on the total volume of the culture medium, and the culture medium contains 10-100 g/L of sucrose based on the total volume of the medium.

The concentration of IBA or NAA may be at least 0.1 mg/L, at least 0.5 mg/L, at least 1 mg/L, at least 2 mg/L, at least 3 mg/L, at least 3.5 mg/L, at least 3.6 mg/L, at least 3.7 mg/L, at least 3.8 mg/L, at least 3.9 mg/L, at least 4 mg/L, or at least 5 mg/L. Further, the concentration of IBA or NAA may be 10 mg/L or less, 9 mg/L or less, 8 mg/L or less, 7 mg/L or less, 6 mg/L or less, 5 mg/L or less, 4.5 mg/L or less, 4.3 mg/L or less, 4.1 mg/L or less, 3.5 mg/L or less or 3 mg/L or less.

According to an aspect, in the step (b), the culture medium contains 2 to 8 mg/L of IBA (indole butyric acid) based on the total volume of the culture medium. Specifically, IBA (indole butyric acid) may be contained at an amount of at least 2 mg/L, at least 3 mg/L, at least 3.6 mg/L, at least 3.7 mg/L, at least 3.8 mg/L, at least 3.9 mg/L, at least 4 mg/L, or at least 5mg/L, and also at an amount of 8 mg/L or less, 7 mg/L or less, 6 mg/L or less, 5 mg/L or less, 4.5 mg/L or less, 4.3 mg/L or less, 4.1 mg/L or less, 3.5 mg/L or less, or 3 mg/L or less based on the total volume of the culture medium.

According to an aspect, the leguminous seeds in the step (a) are from Glycine Max Merr.; the induced site in the step (b) is at least one of hypocotyls and radicles; and the culture medium in the step (b) contains 3 to 5 mg/L of IBA (indole butyric acid) based on the total volume of the culture medium.

According to the method as defined in annexed claim 1, in the step (c), the culture medium contains 3 to 5 mg/L of IBA (indole butyric acid) and 10-100 g/L of sucrose based on the total volume of the culture medium.

According to an aspect, in the step (c), the culture medium is 0.5-1.5 MS medium and contains 30-60 g/L of sucrose.

Specifically, the concentration of sucrose in the step (c) may be at least 10 g/L, at least 20 g/L, at least 21 g/L, at least 22 g/L, at least 23 g/L, at least 24 g/L, at least 25 g/L, at least 26 g/L, at least 27 g/L, at least 28 g/L, at least 29 g/L, or at least 30 g/L based on the total volume of the culture medium. Further, the concentration of sucrose may be 100 g/L or less, 80 g/L or less, 60 g/L or less, 50 g/L or less, 40 g/L or less, 39 g/L or less, 38 g/L or less, 37 g/L or less, 36 g/L or less, 35 g/L or less, 34 g/L or less, 33 g/L or less, 32 g/L or less, 31 g/L, or less or 30 g/L or less based on the total volume of the culture medium. When the concentration of sucrose in the step (c) is within the range indicated above, a high multiplication of cultured roots can be achieved, and the content of coumestrol in cultured roots can be increased.

Moreover, the culture medium in the step (c) may be 0.5 MS, 0.6 MS, 0.7 MS, 0.75 MS, 0.8 MS, 0.9 MS, 1.0 MS, or 1.5 MS medium according to the concentration of inorganic substance in the medium. When MS medium according to the concentration of inorganic substance in the step (c) is at least one of MS media listed above, a high multiplication of cultured roots can be achieved, and the content of coumestrol in cultured roots can be increased.

According to another aspect, the method provides cultured leguminous roots having increased coumestrol content, produced by any one of the aforementioned methods.

According to another aspect, the content of coumestrol in the cultured roots is at least 0.001 wt% with respect to the total dry weight of the cultured roots. Specifically, the content of coumestrol in the cultured roots may be at least 0.001 wt%, at least 0.002 wt%, at least 0.003 wt%, at least 0.004 wt%, at least 0.005 wt%, at least 0.006 wt%, at least 0.007 wt%, at least 0.008 wt%, at least 0.009 wt%, at least 0.01 wt%, at least 0.012 wt%, at least 0.014 wt%, at least 0.015 wt%, at least 0.016 wt%, or at least 0.017 wt% with respect to the total dry weight of the cultured roots. Further, the content of coumestrol in the cultured roots may be 1 wt% or less, 0.9 wt% or less, 0.8 wt% or less, 0.6 wt% or less, 0.5 wt% or less, 0.4 wt% or less, 0.3 wt% or less, or 0.2 wt% or less with respect to the total dry weight of the cultured roots. Cultured roots cultured according to the culturing method for cultured leguminous roots according to the present invention may contain 0.01-0.02 wt% of coumestrol with respect to the total dry weight of the cultured roots.

[Examples]

Hereinafter, the present invention will be described in detail with reference to Examples. It will be obvious to those skilled in the art that these examples are only for illustrating the present invention but are not intended tc limit the scope of the invention.

[Preparation Example 1-1] Germination of bean seeds and *in vitro* induction of plant

Each surface of Glycine Max Merr. and Glycine gracillis seeds was sterilized using 2% sodium hypochlorite for 20 minutes and washed 3 times with sterile water. Then, a plant was induced in 1/2 MS medium (Murashige and Skoog Medium) supplemented with 30 g/L of sucrose under light condition at 25±1 °C for 2-3 weeks.

The procedure of inducing an *in vitro* plant from leguminous seeds is shown in Fig. 1.

[Preparation Example 1-2] Induction of cultured bean roots

Cotyledons, hypocotyls, and radicles from the induced plant were cut to about 1cm and maintained in 1 MS medium supplemented with 30 g/L of sucrose with 0, 1.0, 2.0, 4.0, 8.0mg/L of IBA (indole butyric acid) and NAA (naphthalene acetic acid), respectively, under dark condition at 22±1 °C for 2-3 weeks to induce cultured roots.

The induction status depending on the type of bean, the induced site, and the type and amount of auxin are shown in Figs. 2 and 3

[Experimental Example 1] Measurement of dry substance productivity and coumestrol content of cultured roots depending on the variety of bean, the induced site, and the type and amount of auxin

Dry substance productivity from dry cultured bean roots of the Preparation Example 1-2 was measured. The result is shown in Fig. 4. Growth statuses of cultured bean roots were different depending on the type of bean and the induced site. In particular, the net weight of cultured roots derived from radicles of Glycine Max Merr. was increased by 12.5 times with respect to the initial value after 4 weeks of culture. The final dry substance productivity was also excellent as 4.1 g/L.

Further, the dry cultured bean roots of Preparation Example 1-2 were extracted in 80% (w/v) ethanol at room temperature for 24 hours. The resulting extract was filtered using a filter paper and dried by evaporating a solvent to obtain a powder. Then, the powder was diluted to be a 1% solution to prepare a final extract. To measure the content of coumestrol in the cultured roots, 2 mL of the extract was filtered with 0.45 μm filter and injected into a high-performance liquid chromatography with a UV detector by 10 μL. A column of Mightysil RP-18 GP 250-4.6 (5 μm) column was used. The content of coumestrol in the extract was measured at 342 nm wavelength. The result is shown in Fig. 5. As seen in Fig. 5, the coumestrol content was the highest as 0.18 mg/g DW in the cultured roots derived from radicles of Glycine Max Merr.

Based on the experimental results indicated above, the cultured roots derived from radicles of Glycine Max Merr. having the highest coumestrol content were multiplied using the method in Preparation Example 1-3 as described below.

[Preparation Example 1-3] Optimum medium condition for multiplication of cultured bean roots using DOE method and multiplication using said medium

When carrying out multiplication in a bioreactor, the medium composition suitable for coumestrol production may be varied depending on the medium condition (concentrations of inorganic substance and sucrose). Thus, using cultured roots derived from radicles of Glycine Max Merr. which is determined to be most suitable for producing coumestrol, an optimum medium composition was selected by DOE method, and the cultured roots were multiplied in the selected medium.

(1) Selection of optimum medium composition for multiplication of cultured roots

By using Design of Experiments (DOE) method, an experiment for optimizing a medium composition was performed. The condition of the experiment was as follows:

2 factors X 3 levels X 2 repetitions (treatment factor: sucrose X MS Salt / treatment level: sucrose = 1-9%/MS Salt = 0.25-2 times)

The result from the prediction models obtained according to said method is shown in Fig. 6. From this result, it was demonstrated that the optimum medium composition has 0.5-1 MS of inorganic substance and 3~6% of sucrose in the medium.

(2) Multiplication of cultured roots in the optimum medium

The cultured roots of Preparation Example 1-2 were multiplied at 3 to 4-week intervals using 2L of 1 MS medium with 4 mg/L of IBA and 30 g/L of sucrose in a bulb type bioreactor with 3L air volume. The medium was used after adjusting the pH to 5.8 using 1N NaOH and sterilizing at 121°C and 1.2 atmospheric pressure for 35 minutes. The cultured roots were cultured by cutting them to 1 to 1.5cm, inoculating the cultured roots at an inoculation density of 4-5 g/L with respect to its net weight, and culturing under dark condition at 22±1 °C. Air was supplied at a constant amount of 0.1 vvm throughout the culture process. To maintain the temperature temperature of air provided in the bioreactor constant, air was sequentially passed through an air compressor capable of condensing compressed air, a filter capable of removing impurities, and an air drier, and then supplied to the bioreactor using an oil-free air compressor.

[Experimental Example 2] Measurement of coumestrol content in multiplied bean roots

The content of coumestrol was measured on bean roots multiplied in Preparation Example 1-3 using the same method as in Experimental Example 1.

The coumestrol content was measured to be 0.16 mg/g dry weight (0.016 wt%) with respect to the dry weight of the multiplied bean roots.

While the present invention has been described with reference to specific embodiments thereof, but it is to be understood by a person skilled in the art that these specific embodiments are merely preferred embodiments and that the scope of the present invention is not limited thereby. Accordingly, the scope of the present invention will be defined by the appended claims.

## Claims

1. A culturing method for cultured leguminous roots having increased coumestrol content comprising the steps of:

   (a) germinating seeds of leguminous plant in a culture medium to induce *in vitro* plants having hypocotyls and radicles;
   (b) culturing, in a culture medium, at least one site of hypocotyls, and radicles of the induced *in vitro* plant to induce cultured roots derived from at least one site of hypocotyls and radicles; and
   (c) multiplying the induced cultured roots derived from at least one site of hypocotyls and radicles in a culture medium;
   wherein the culture medium contains nutrient components of $NH_4NO_3$, $CaCl_2•2H_2O$, $MgSO_4•7H_2O$, $KH_2PO_4$, and $KNO_3$,
   wherein the leguminous plant is Shinhwa bean (*Glycine max* merr.),
   wherein the culture medium is MS medium (Murashige and Skoog medium),
   wherein in the step (a), the culture medium contains 10-100 g/L of sucrose,
   wherein in the step (b), the culture medium contains at least one of indole butyric acid (IBA) and naphthalene acetic acid (NAA) at an amount between 0.1 to 10 mg/L, and the culture medium contains 10-100 g/L of sucrose, and
   wherein in the step (c), the culture medium contains 3 to 5 mg/L of IBA (indole butyric acid) and 10-100 g/L of sucrose.

2. The culturing method of claim 1, wherein the concentration of $NH_4NO_3$ in the culture medium is between 1,500 and 2,000 mg/L, the concentration of $CaCl_2•2H_2O$ in the culture medium is between 300 and 500 mg/L, the concentration of $MgSO_4•7H_2O$ in the culture medium is between 300 and 500 mg/L, the concentration of $KH_2PO_4$ in the culture medium is between 100 and 200 mg/L, and the concentration of $KNO_3$ in the culture medium is between 1,700 and 2,100 mg/L.

3. The culturing method of claim 1, wherein in the step (b), the culture medium contains 2 to 8 mg/L of indole butyric acid (IBA).

4. The culturing method of claim 1, wherein the culture medium in the step (b) contains 3 to 5 mg/L of indole butyric acid (IBA).

5. The culturing method of claim 1, wherein in the step (c), the culture medium is 0.5-1.5 MS medium and contains 30-60 g/L of sucrose.

## Patentansprüche

1. Verfahren zur Kultivierung von kultivierten Wurzeln von Hülsenfrüchten mit erhöhtem Coumestrolgehalt, umfassend die folgenden Schritte:

(a) Keimen von Samen einer Hülsenfruchtpflanzein einem Kulturmedium, um *In-vitro*-Pflanzen mit Hypokotylen und Keimwurzeln zu induzieren;
(b) Kultivieren mindestens einer Stelle von Hypokotylen und Keimwurzeln der induzierten In-vitro-Pflanze in einem Kulturmedium, um kultivierte Wurzeln zu induzieren, die von mindestens einer Stelle von Hypokotylen und Keimwurzeln stammen; und
(c) Vermehrung der induzierten kultivierten Wurzeln, die von mindestens einer Stelle von Hypokotylen und Keimwurzeln stammen, in einem Kulturmedium;
wobei das Kulturmedium Nährstoffkomponenten von $NH_4NO_3$, $CaCl_2 \cdot 2H_2O$, $MgSO_4 \cdot 7H_2O$, $KH_2PO_4$ und $KNO_3$ enthält,
wobei die Hülsenfrucht Shinhwa-Bohne (*Glycine max* merr.) ist,
wobei das Kulturmedium ein MS-Medium (Murashige- und Skoog-Medium) ist,
wobei in Schritt (a) das Kulturmedium 10-100 g/L Saccharose enthält,
wobei in Schritt (b) das Kulturmedium mindestens eine von Indolbuttersäure (IBA) und Naphthalinessigsäure (NAA) in einer Menge zwischen 0,1 und 10 mg/L enthält und das Kulturmedium 10 bis 100 g/L Saccharose enthält, und
wobei in Schritt (c) das Kulturmedium 3 bis 5 mg/L IBA (Indolbuttersäure) und 10 bis 100 g/L Saccharose enthält.

**2.** Das Kultivierungsverfahren nach Anspruch 1, wobei die Konzentration von $NH_4NO_3$ im Kulturmedium zwischen 1.500 und 2.000 mg/L liegt, die Konzentration von $CaCl_2 \cdot 2H_2O$ im Kulturmedium zwischen 300 und 500 mg/L liegt, die Konzentration von $MgSO_4 \cdot 7H_2O$ im Kulturmedium zwischen 300 und 500 mg/L liegt, die Konzentration von $KH_2PO_4$ im Kulturmedium zwischen 100 und 200 mg/L liegt und die Konzentration von $KNO_3$ im Kulturmedium zwischen 1.700 und 2.100 mg/L liegt.

**3.** Das Kultivierungsverfahren nach Anspruch 1, wobei in Schritt (b) das Kulturmedium 2 bis 8 mg/L Indolbuttersäure (IBA) enthält.

**4.** Das Kultivierungsverfahren nach Anspruch 1, wobei das Kulturmedium in Schritt (b) 3 bis 5 mg/L Indolbuttersäure (IBA) enthält.

**5.** Das Kultivierungsverfahren nach Anspruch 1, wobei in Schritt (c) das Kulturmedium ein 0,5-1,5 MS-Medium ist und 30-60 g/L Saccharose enthält.

## Revendications

**1.** Procédé de culture de racines de légumineuses cultivées ayant une teneur accrue en coumestrol, comprenant les étapes suivantes :

(a) faire germer des graines de plante légumineuse dans un milieu de culture afin d'induire des plantes *in vitro* ayant des hypocotyles et des radicelles ;
(b) cultiver, dans un milieu de culture, d'au moins un site d'hypocotyles et de radicelles de la plante *in vitro* induite afin d'induire des racines cultivées dérivées d'au moins un site d'hypocotyles et de radicelles ; et
(c) multiplier les racines en culture induites dérivées d'au moins un site d'hypocotyles et de radicelles dans un milieu de culture ;
dans lequel le milieu de culture contient des composants nutritifs de $NH_4 NO_3$, $CaCl_2 \cdot 2H_2O$, $MgSO_4 \cdot 7H_2O$, $KH_2PO_4$ et $KNO_3$,
dans lequel la plante légumineuse est le haricot Shinhwa (*Glycine max* merr.),
dans lequel le milieu de culture est un milieu MS (milieu Murashige et Skoog),
dans lequel, dans l'étape (a), le milieu de culture contient 10 à 100 g/L de saccharose,
dans lequel, dans l'étape (b), le milieu de culture contient au moins l'un des composés suivants : l'acide indolebutyrique (IBA) et l'acide naphtalèneacétique (NAA) en une quantité comprise entre 0,1 et 10 mg/L, et le milieu de culture contient 10 à 100 g/L de saccharose, et
dans lequel, dans l'étape (c), le milieu de culture contient 3 à 5 mg/L d'IBA (acide indole butyrique) et 10 à 100 g/L de saccharose.

**2.** Procédé de culture selon la revendication 1, dans lequel la concentration en $NH_4NO_3$ dans le milieu de culture est comprise entre 1 500 et 2 000 mg/L, la concentration en $CaCl_2 \cdot 2H_2 O$ dans le milieu de culture est comprise entre 300 et 500 mg/L, la concentration en $MgSO_4 \cdot 7H_2 O$ dans le milieu de culture est comprise entre 300 et 500 mg/L, la

concentration de $KH_2PO_4$ dans le milieu de culture est comprise entre 100 et 200 mg/L, et la concentration de $KNO_3$ dans le milieu de culture est comprise entre 1 700 et 2 100 mg/L.

**3.** Procédé de culture selon la revendication 1, dans lequel, à l'étape (b), le milieu de culture contient 2 à 8 mg/L d'acide indole butyrique (IBA).

**4.** Procédé de culture selon la revendication 1, dans lequel le milieu de culture à l'étape (b) contient 3 à 5 mg/L d'acide indole butyrique (IBA).

**5.** Procédé de culture selon la revendication 1, dans lequel, à l'étape (c), le milieu de culture est un milieu MS 0,5-1,5 et contient 30 à 60 g/L de saccharose.

FIG. 1

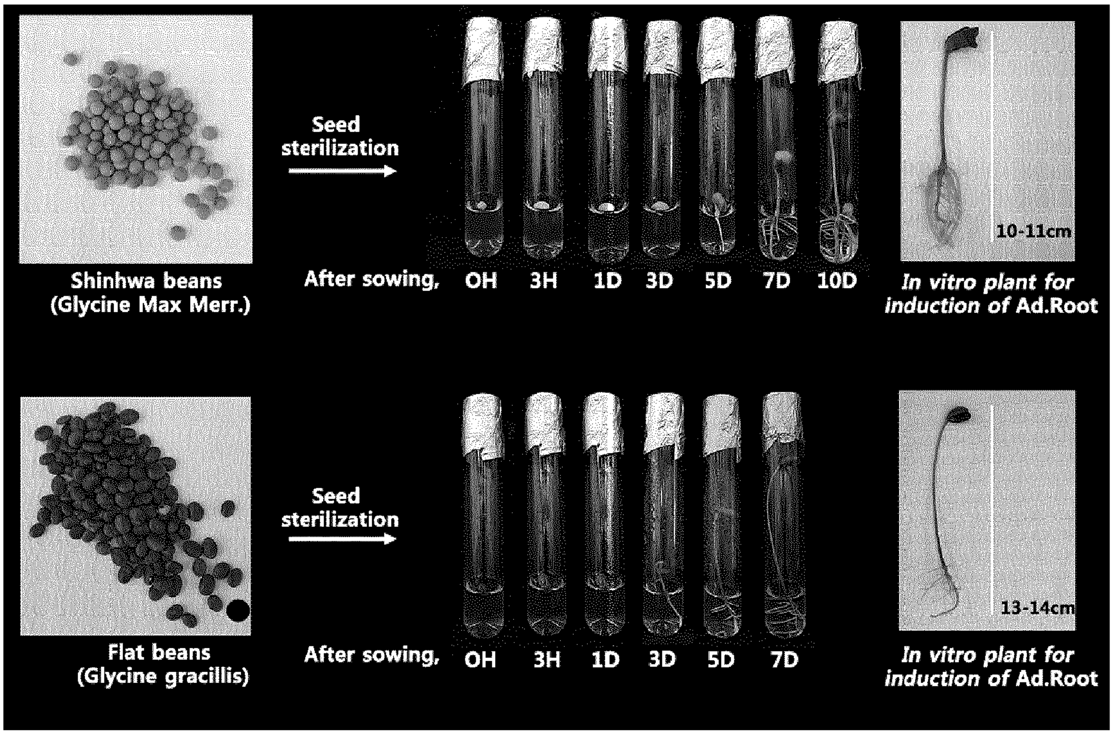
Shinhwa beans
(Glycine Max Merr.)
Seed
sterilization
After sowing,
OH
3H
1D
3D
5D
7D
10D
10-11cm
In vitro plant for
induction of Ad.Root
Flat beans
(Glycine gracillis)
Seed
sterilization
After sowing,
OH
3H
1D
3D
5D
7D
13-14cm
In vitro plant for
induction of Ad.Root

FIG. 2

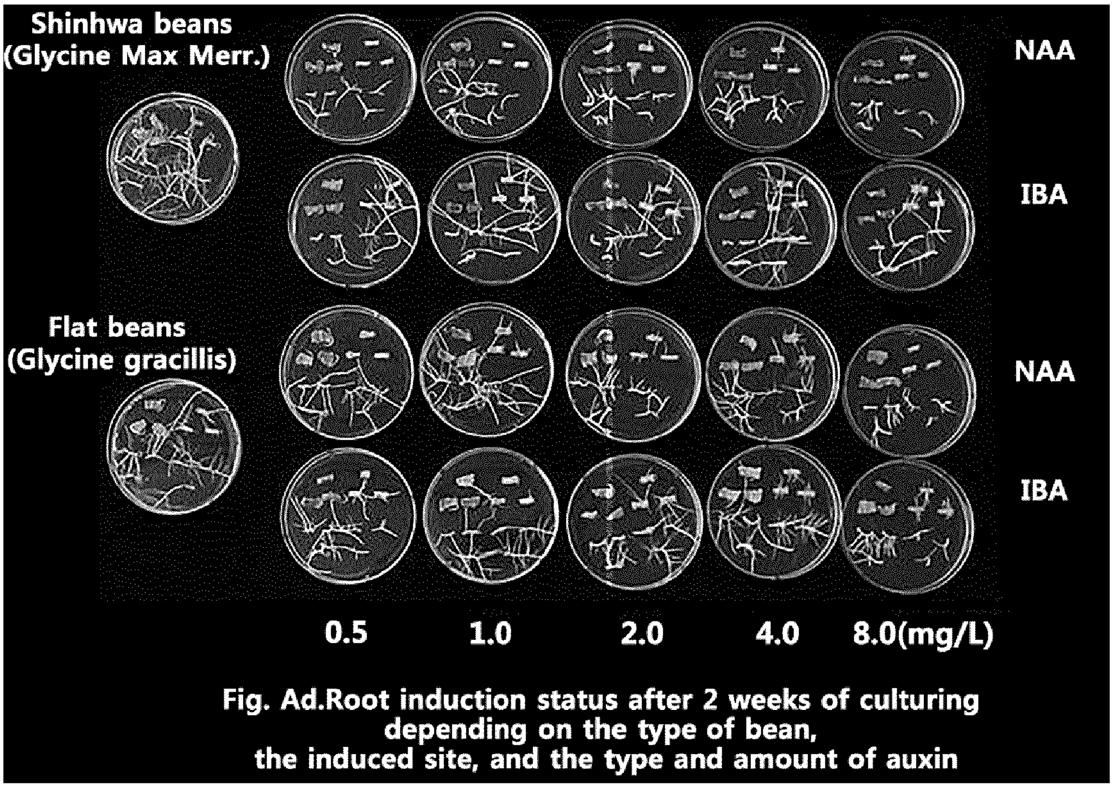
Shinhwa beans
(Glycine Max Merr.)
Flat beans
(Glycine gracillis)
NAA
IBA
NAA
IBA
0.5
1.0
2.0
4.0
8.0(mg/L)
Fig. Ad.Root induction status after 2 weeks of culturing depending on the type of bean, the induced site, and the type and amount of auxin

FIG. 3

**Table. Ad.Root induction status after 2 weeks of culturing depending on the type of bean, the induced site, and the type and amount of auxin**

| Species | Auxin | Explant types | Conc. (mg/L) | | | | | |
|---|---|---|---|---|---|---|---|---|
| | | | 0 | 0.5 | 1.0 | 2.0 | 4.0 | 8.0 |
| Glycine Max Merr. | NAA | Cotyledon | | | | - | - | |
| | | Hypocotyl | - | | | + | + | + |
| | | Axial root | ++ | - | ++ | ++ | ++ | + |
| | | Side root | ++ | - | + | + | + | - |
| | IBA | Cotyledon | | - | - | - | - | |
| | | Hypocotyl | - | - | + | ++ | ++ | ++ |
| | | Axial root | ++ | + | + | + | - | - |
| | | Side root | ++ | ++ | ++ | ++ | ++ | ++ |
| Glycine gracillis | NAA | Cotyledon | | | | - | - | - |
| | | Hypocotyl | - | - | + | + | ++ | - |
| | | Axial root | ++ | ++ | +++ | +++ | +++ | ++ |
| | | Side root | ++ | ++ | ++ | + | + | - |
| | IBA | Cotyledon | | | | - | + | - |
| | | Hypocotyl | - | - | - | ++ | +++ | +++ |
| | | Axial root | ++ | ++ | ++ | ++ | +++ | ++ |
| | | Side root | ++ | + | ++ | +++ | +++ | - |

+++ very good, ++ good, + normal, - not good

FIG. 4

**Table. Growth amount of cultured roots after 4 weeks of culturing depending on the type of bean and the induced site**

| Genotypes | Explant sources | Fresh weight (g/L) | Dry weight (g/L) | % Dry weight |
|---|---|---|---|---|
| Glycine Max Merr. | Hypocotyl | 33.9±1.20 | 3.0±0.11 | 8.8±0.07 |
| | Radicle | 50.3±2.09 | 4.1±0.17 | 8.2±0.06 |
| Glycine gracillis | Hypocotyl | 16.4±0.46 | 1.6±0.03 | 9.7±0.08 |
| | Radicle | 12.5±0.64 | 1.3±0.06 | 10.4±0.06 |

※ Culturing condition
- Medium composition: 1 MS + IBA 4mg/L + Suc. 3%
- Culturing temperature: 22±1 °C (dark condition)
- Culturing period: 4 weeks
- Culturing container = 3L Bulb Type Bioreactor (Working vol. 2L)
- Inoculating density = 4g/L Wet Weight

FIG. 5

**Table. Content of bioactive substances in cultured roots after 4 weeks culturing depending on the type of bean and the induced site**

| Geno types | Explant Sources | Yield (%) | Coumestrol | |
|---|---|---|---|---|
| | | | (mg/g DW) | Total production (mg/L) |
| Glycine Max Merr. | *Ex vitro* roots | 2.9±0.07 | 0.01±0.000 | - |
| | Hypocotyl | 31.0±0.67 | 0.17±0.006 | 0.49±0.017 |
| | Radicle | 34.8±0.64 | 0.18±0.013 | 0.75±0.055 |
| Glycine gracillis | *Ex vitro* roots | 7.0±0.07 | 0.01±0.000 | - |
| | Hypocotyl | 25.1±0.44 | 0.16±0.010 | 0.26±0.015 |
| | Radicle | 24.5±0.14 | 0.17±0.019 | 0.23±0.024 |

※ Total Production (mg/L)
= Average dry substance of each treatment group (mg/L) X Average content of each bioactive substance (mg/g DW)

※ Ex vitro roots
= Harvest of Oct. 19, 2016 (R8/seed maturation stage)

FIG. 6

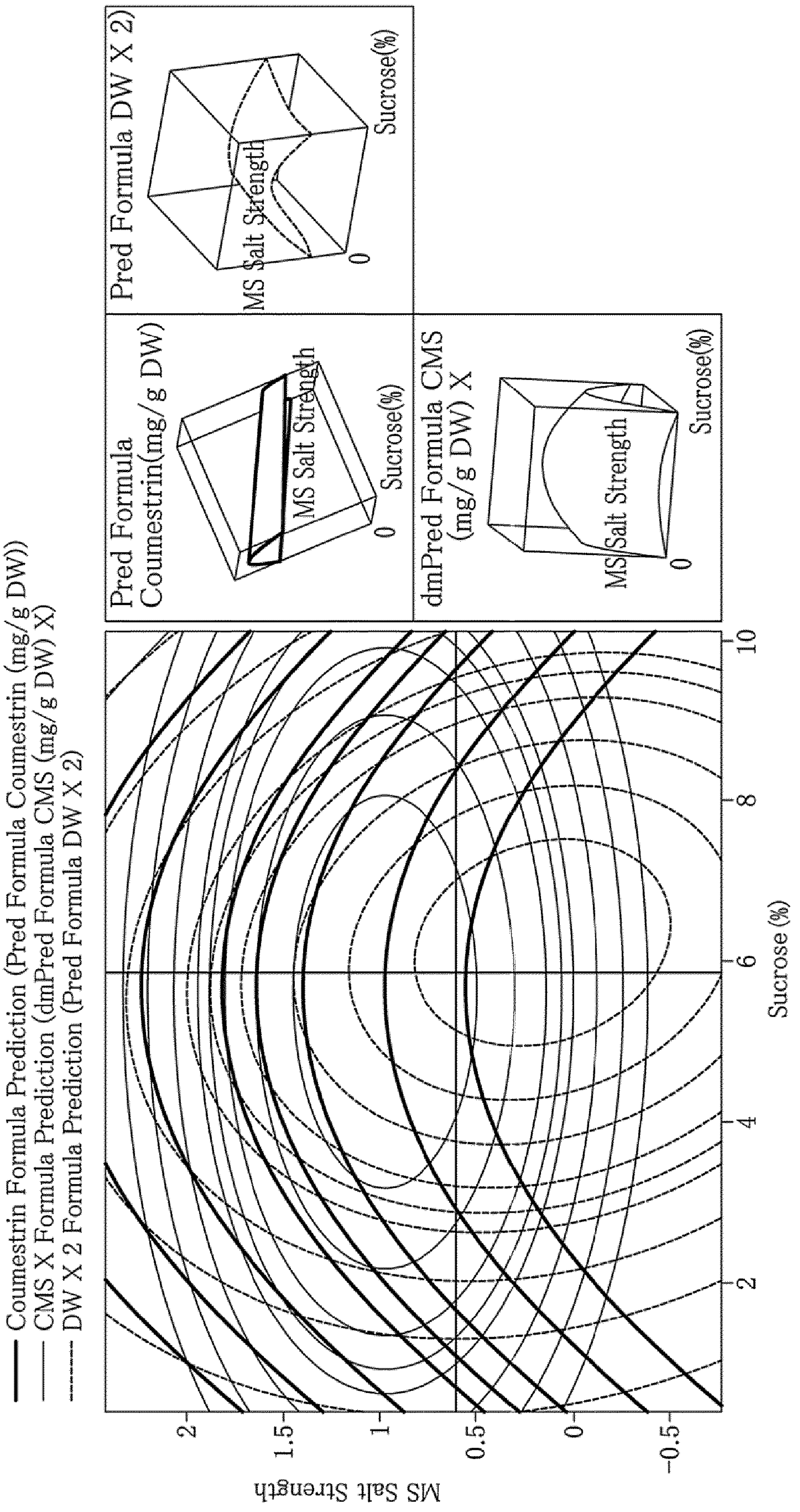
Coumestrin Formula Prediction (Pred Formula Coumestrin (mg/g DW))
CMS X Formula Prediction (dmPred Formula CMS (mg/g DW) X)
DW X 2 Formula Prediction (Pred Formula DW X 2)
Pred Formula Coumestrin(mg/g DW)
MS Salt Strength
Sucrose(%)
0
Pred Formula DW X 2
MS Salt Strength
Sucrose(%)
0
dmPred Formula CMS (mg/g DW) X
MS Salt Strength
Sucrose(%)
0
MS Salt Strength
2
1.5
1
0.5
0
−0.5
Sucrose (%)
2
4
6
8
10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 20140060201 A


### Non-patent literature cited in the description


- Psoralen production in hairy roots and adventitious roots cultures of Psoralea coryfolia. **BASKARAN P et al.** BIOTECHNOLOGY LETTERS. SPRINGER NETHERLANDS, 07 March 2009, vol. 531, 1073-1077
- **BOURGAUD, F. et al.** Production of Flavonoids by Psoralea Hairy Root Cultures. *PLANT CELL, TISSUE AND ORGAN CULTURE*, 1999, vol. 56, 97-104
- **SOO HA et al.** Adventitious root formation from cotyledon in soybean (Glycine max L.) cultivars. *Korean J. Plant Biotechnology*, 01 January 2002, vol. 29, 31-36
- Effect of PGRs in adventitious root culture in vitro: present scenario and future prospects. **AHMAD NASEEM et al.** RENDICONTI LINCEI. SCIENZE FISICHE E NATURALI. SPRINGER ITALIA SRL, 23 June 2015, vol. 26, 307-321
- **YUN SUN LEE et al.** Induction and Proliferation of Adventitious Roots from 'Aloe vera' Leaf Tissues for 'in vitro' Production of Aloe-emodin. *Plant Omics*, 01 July 2011, 190-194
- **MAGDALENA WÓJCIAK-KOSIOR et al.** The Stimulatory Effect of Strontium Ions on Phytoestrogens Content in Glycine max (L.) Merr. *MOLECULES*, 14 January 2016, vol. 21 (1), 90